Europäisches Patentamt

(19) European Patent Office

Office européen des brevets

(11) Publication number: **0 126 019**

**A2**

# (12) EUROPEAN PATENT APPLICATION

(21) Application number: **84630076.2**

(22) Date of filing: **15.05.84**

(51) Int. Cl.³: **C 12 Q 1/24**
**C 12 Q 1/04**

(30) Priority: **16.05.83 US 495206**

(43) Date of publication of application:
**21.11.84 Bulletin 84/47**

(84) Designated Contracting States:
**CH DE FR GB IT LI SE**

(71) Applicant: **Packard Instrument Company, Inc.**
**2200 Warrenville Road**
**Downers Grove Illinois(US)**

(72) Inventor: **Everett, Leroy J.**
**275 South Milton Avenue**
**Glen Ellyn Illinois 60137(US)**

(72) Inventor: **Tsai, TenLin Sie**
**7130 Summit Road**
**Darien Illinois 60559(US)**

(72) Inventor: **Ramey, Norma J.**
**Northwest 180 204 West Oakley Drive**
**Westmont Illinois 60559(US)**

(74) Representative: **Schmitz, Jean-Marie et al,**
**Office Dennemeyer S.à.r.l. 21-25 Allée Scheffer P.O. Box 41**
**L-2010 Luxembourg(LU)**

(54) **High resolution method of measuring ATP, and concentrating and measuring unicellular organisms.**

㊒ A method of concentrating and measuring the amount of cells in a sample is described. A sample containing the plurality of cells is passed upwardly or laterally through a series of membrane filters and concentrated on the filter. The filter membrane containing the concentrated cell is then sequentially treated with lysing agent to release the adenosine triphosphate from the organism and a luminescent reagent to react with the released adenosine triphosphate. A luminescent response is thereby produced directly on the filter membrane and this luminescent response is measured with a luminometer. Not only does this method provide an accurate way of detecting very minute quantities of cells but it provides a relatively quick and easy method of concentrating and measuring cells in large volumes of biological or industrial samples which contain low levels of organisms. This method can also be used to identify and measure sources of adenosine triphosphate. Another aspect of the disclosed method is the utilization of a series of filters, each of which traps and concentrates cells of different size. For example, a sample containing a plurality of cell types can be passed through a series of filter membranes, with different pore size each of which allows passage of all but one unicellular organism, resulting in a concentration of different cells on different filter membranes. The filter membranes can then be processed and measured on the luminometer as described above.

./...

FIG.1

MIXED PURE CULTURE - YEAST, E. COLI, P. AERUGINOSA, S. AUREUS - DOUBLE FILTER FILTRATION

LIGHT RESPONSE
(COUNTS/30 SEC)
ON
0.4 MICRON FILTER

MIXED BACTERIA CULTURE (CFU/10 ML)

### High Resolution Method of Measuring ATP and Concentrating and Measuring Unicellular Organisms

Technical Field

The field of art to which this invention pertains is assay methods, and particularly methods of measuring ATP and unicellular organisms.

Background Art

Quantitative determinations of the presence of adenosine triphosphate (ATP) and unicellular organisms such as bacteria, yeast cells, and white blood cells are important in many areas. For example, the detection of the presence of bacteria in biological fluids, food products, industrial and environmental samples is important. If bacteria levels exceed a certain maximum, the economic damage can be devastating. Similarly, the detection of yeast levels in beverages and dairy products can simplify the monitoring of the stages of fermentation and is vital to the successful use of these products. Conventional methods useful for detecting the presence and/or amount of such cells in a particular area are generally time consuming, relatively tedious and not very accurate, especially when the cell concentrations are less than about $1 \times 10^5$ cells per milliliter. Furthermore, in detecting for a plurality of types of cells, the process of detection is compounded significantly.

Accordingly, what is needed in this art is a simple method of detecting ATP and unicellular organisms even in relatively low concentrations, and

JP-1594

even for a sample containing a plurality of types of organisms.

Disclosure of Invention

A method of measuring the concentration of adenosine triphosphate containing unicellular organisms in a sample is described. The sample containing the organisms is passed upwardly through a series of filter membranes of such relative pore sizes as to concentrate the organism on the desired filter membrane. The upward filtration keeps the organism in suspension, thus avoiding precipitation of any organisms resulting in a highly accurate organism count. The upward filtration and the use of a plurality of filters also has the advantage of removing unwanted components from the concentrating filter such as interfering physical debris and chemical particles. Filter clogging is also prevented.

Another aspect of the invention includes sizing the plurality of filters to selectively concentrate a variety of organisms present in one sample pass. For example, three separate types of cells contained in one sample can be passed through three spaced apart filters of differing pore size where one of each of the organisms is impermeable to at least one of the filters where it is collected and concentrated.

Another aspect of the invention includes a method of separating microbial adenosine triphosphate (ATP), non-microbial ATP and extra-cellular ATP. This is accomplished by passing a sample containing the various forms of ATP upwards through a series of filter membranes of decreasing pore size. The pores of the filters are sized so as to allow passage of the extra-cellular ATP which may be collected in the filtrate. At least one filter membrane is selected with pore size impermeable to cells containing ATP, and at least one filter membrane is selected with a pore size to be impermeable to cells containing non-microbial ATP. The filter membrane impermeable to the non-microbial ATP is placed upstream of the filter membrane impermeable to the microbial ATP. This provides a useful method for collecting, measuring and identifying sources of ATP.

Another aspect of the invention includes a method of cell enrichment of a specific cell type. A sample containing a plurality of cell types is passed in upward manner through a series of filter membranes of decreasing pore size of which at least one filter membrane is impermeable to the specific cell type desired to be enriched.

Another aspect of the invention includes a method of isolating a sample of substantially pure bacteria cells from a sample containing soluble interferences which would adversely interfere with an accurate count of such cells. An upward filtration method is used here which allows passage of the interferences through a filter membrane selected of such pore size as to trap and concentrate the bacteria cells desired to be collected.

After concentrating on the respective filters, the filter membranes containing the concentrated organisms are sequentially treated with a lysing agent and luminescent reagent. The lysing agent lyses the cell releasing the adenosine triphosphate (ATP) present in the cells. The ATP is then reacted with the luminescent reagent producing a luminescent response (light) which is measured on a luminometer.

The foregoing, and other features and advantages of the present invention, will become more apparent from the following description and accompanying drawing.

Brief Description of the Drawings

Fig. 1 shows the light response for a mixed bacteria culture on a polycarbonate, 13 millimeter diameter, 0.4 micron pore size membrane filter culture.

Fig. 2 shows light response for a mixed E. coli and yeast culture.

Fig. 3 shows light response of a ground beef sample.

Fig. 4 shows light response for a spoiled ground beef sample.

Fig. 5 shows light response for a skim milk sample.

Fig. 6 shows light response for a fish sample.

Fig. 7 shows light response of a human urine sample.

Best Mode for Carrying Out the Invention

As stated above one of the advantages of the method of the present invention is a way to keep the unicellular organisms in suspension and eliminate precipitation of organisms thus resulting in a more accurate count. Other advantages include removal of extraneous material and the ability to detect multiple organisms in a single sample.

The method of the present invention includes the use of at least two filters including two filter holders with the larger diameter and larger pore size filter being upstream (direction of flow from large to small pore size) of the second filter. Typically, a Swinney filter holder housing a polycarbonate filter of 13 millimeter diameter and 0.4 micron pore size is connected to a 25 millimeter filter holder housing a 25 millimeter diameter and 5 micron pore size filter.

Aqueous samples are taken into a sterile syringe and pushed by syringe pump upward at a rate approximately 2.5 ml/minute, to the sequentially connected 25 millimeter filter followed by passage through the 13 millimeter filter. The end filtrate may be typically collected in a sterile vessel.

The first filter retains large particulate or larger cells (such as mammalian cells and/or yeast) and bacteria cells, for example, in the sample will pass through the first filter of larger pore size and be concentrated on the second filter. Free ATP from broken cells and other sources and soluble interferences such as color, antibiotics, chemicals, etc., will pass through both filters and end up in the end filtrate, thus resulting in a more accurate cell ATP measurement. The starting sample and the end filtrate can be comparatively assayed by plating on agar to assure that no bacterial cells escape the smaller pore size filter.

While the invention is described in terms of detecting the numbers of cells on a luminometer, this method may similarly be used for simple separation of the cells for other experimentation. The cells retained on the filters (the 13 millimeter and/or 25 millimeter) can be cultured with rich nutrient sources for enrichment of cells and further identification of cultures of cells or lysing and treatment with luminescent reagent can be done. A T-connector can also be inserted between the various filter holders and samples of the filtrate diverted for different analyses.

The luminescence flash response can be obtained on any conventionally used polymeric filter membranes with a porosity sufficient to trap and collect unicellular organisms (cells) such as polycarbonate, mixed esters of cellulose, cellulose acetate, and Teflon® (du Pont de Nemours & Co., Inc.) membranes. Polycarbonate membranes available from Nuclepore® Corporation, Pleasanton, California, for concentrating cells are preferred because of their pliability and durability.

In use, the sample to be tested for unicellular organisms is filtered directly on conventional filtering equipment if it is in liquid form, or if the sample is in solid form, the cells can be conventionally extracted with such things as buffer solution or water. It should be noted that while descriptions herein are made in terms of cells, actual measurements would be in terms of colony forming units (CFU) which are roughly equivalent.

Filtering equipment typically used includes a plurality of filters housed in 13 mm or 25 mm filter holders connected to a 1 cc to 50 cc sterile disposable syringe. The membranes containing the concentrated cells are generally inserted in a suitable container for bioluminescent determination.

Sufficient lysing reagent should be used to lyse all of the cells in the sample being tested. While most conventional biological cell lysing methods such as boiling Tris (Tris (hydroxymethyl) aminomethane hydrochloride) can be used to lyse the cells and release the cellular ATP, quaternary ammonium salt based PICOEX$^{TM}$ B (Packard Instrument Co., Downers Grove, Illinois) is preferably used with the present invention. A minimum of 100 microliters of PICOEX B added to the filter for a minimum of 2 minutes at room temperature has been found to be optimal for complete lysis of the cells.

Once the cells have been lysed and the ATP released, the luminescent reagent is added to initiate the luminescence response. This response is integrated for a minimum of 15 seconds on a luminometer. While any luminescence reagent may be used, firefly luciferase-luciferin based PICOZYME$^{TM}$ F (Packard Instrument Co.) is preferebly used with the present invention. Here again, sufficient luminescent reagent is added to react with all of ATP released from the cells collected on the filter membrane. For example, a minimum of 100 microliters of PICOZYME F has been found to be optimal for a 13 mm or 25 mm diameter filter membrane.

While any luminometer may be used with the present invention, PICOLITE$^{TM}$ luminometers (Packard Instrument Co.) have been found to be particularly suitable. Utilizing the PICOZYME F with the PICOLITE luminometer, one photon of light is theoretically produced for each ATP molecule reacted.

Utilizing the multiple filter filtration technique described mixed pure culture, meat samples, milk sample and a urine sample were tested for correlation of light response obtained by flashing the membrane filter to the number of cells in the filtration sample.

## Example 1

Enumeration of P. aeruginosa cells are studied by either conventional agar plate count technique or by multiple filtration procedure. The results of the correlation are shown in Table 1.

Table 1

| Filtration Sample (10 ml) | Light Response on 0.4 micron Filter (cts/30 sec) |
|---|---|
| Butterfield's buffer (B.B.) | 11,922 |
| 500 CFU/10 ml | 20,916 |
| 5000 CFU/10 ml | 112,217 |

## Example 2

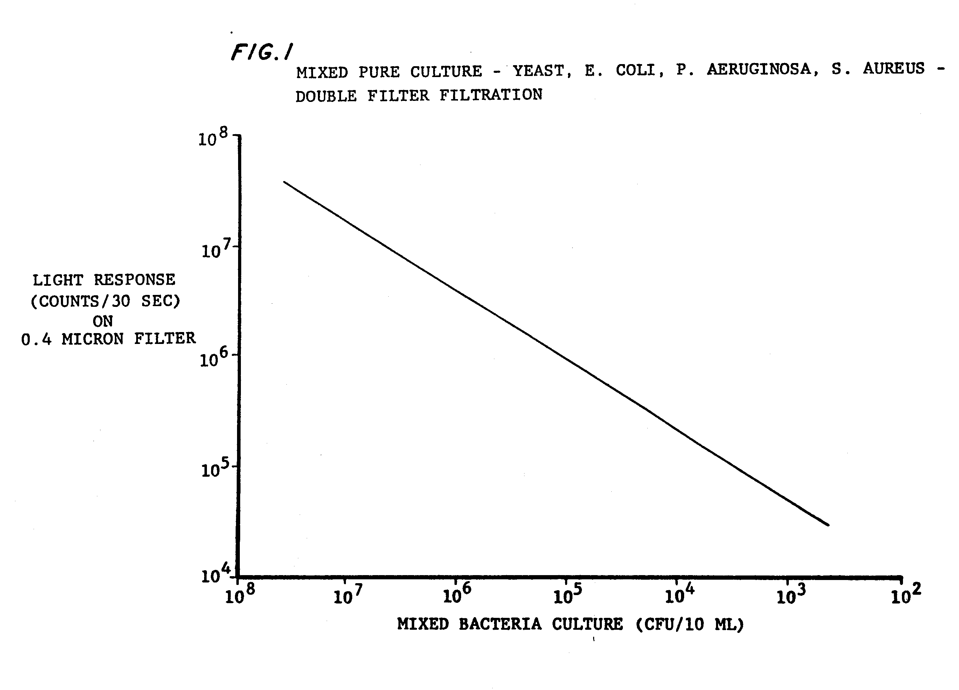

Ten milliliters of serial dilutions of a mixture of the pure cultures including several types of bacterial cells and yeast were put through the multiple filtration system of the present invention. The number of bacteria cells in the sample is lineraly related to the light flash on the 0.4 micron filter as shown in Fig. 1. From observing the filter after incubation on agar plates, most of the yeast culture was stopped by the first five micron pore filter.

## Example 3

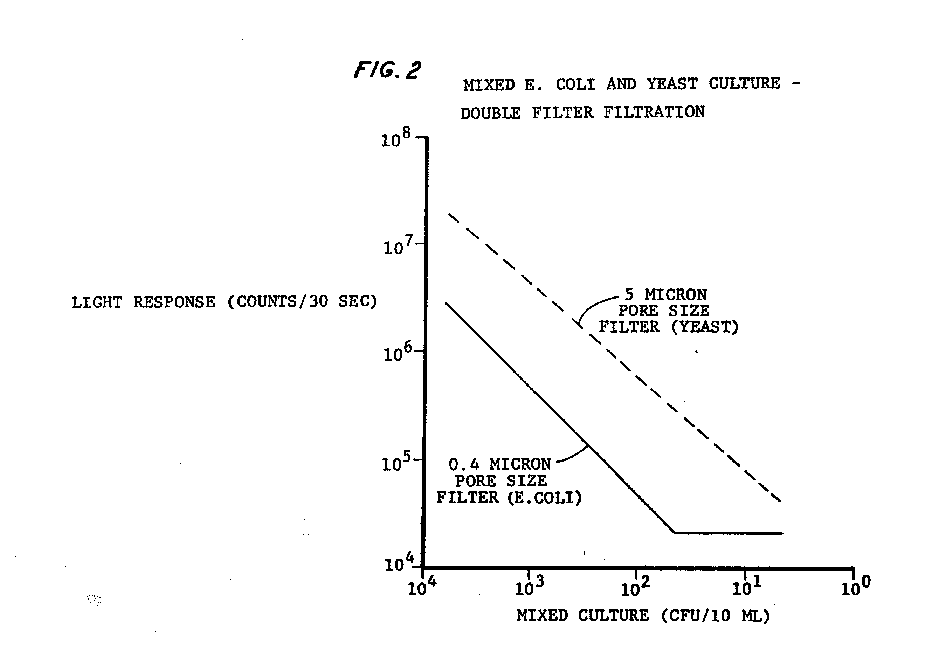

In this experiment the filtration sample contained only E. coli and yeast and the five micron filters are also flashed in order to quantitate the organism retained on the filter, presumably yeast. The two parallel lines shown in Fig. 2 represent light response obtained from the two filters respectively with increasing cells in the sample. The E. coli was concentrated on the 0.4 size micron filter and most of the yeast was concentrated on the 5 micron pore size filter.

## Example 4

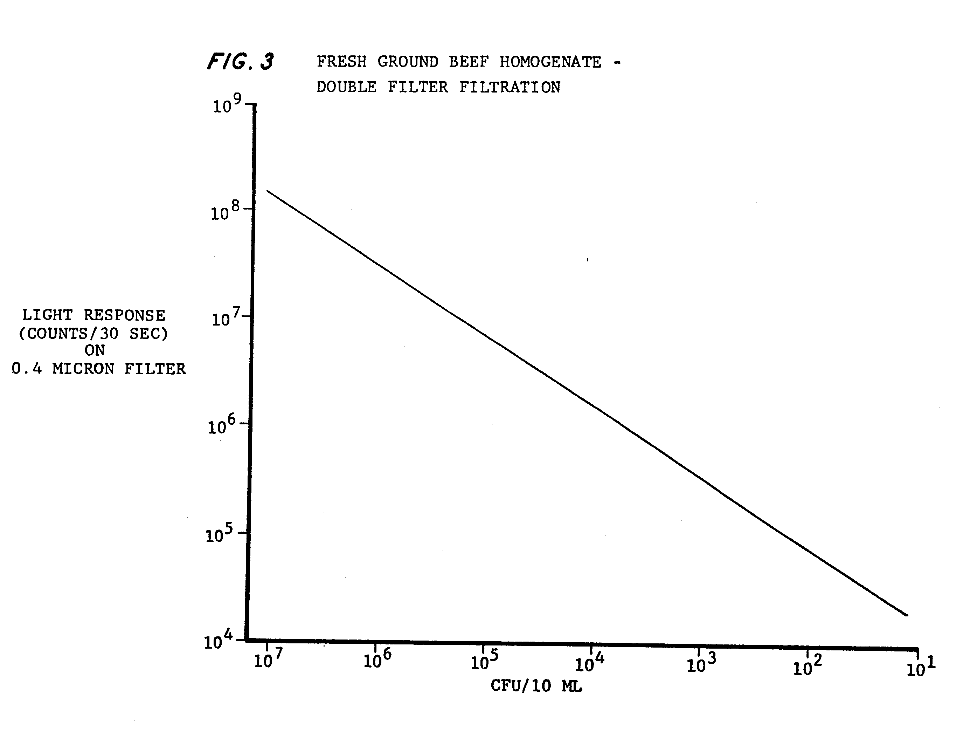

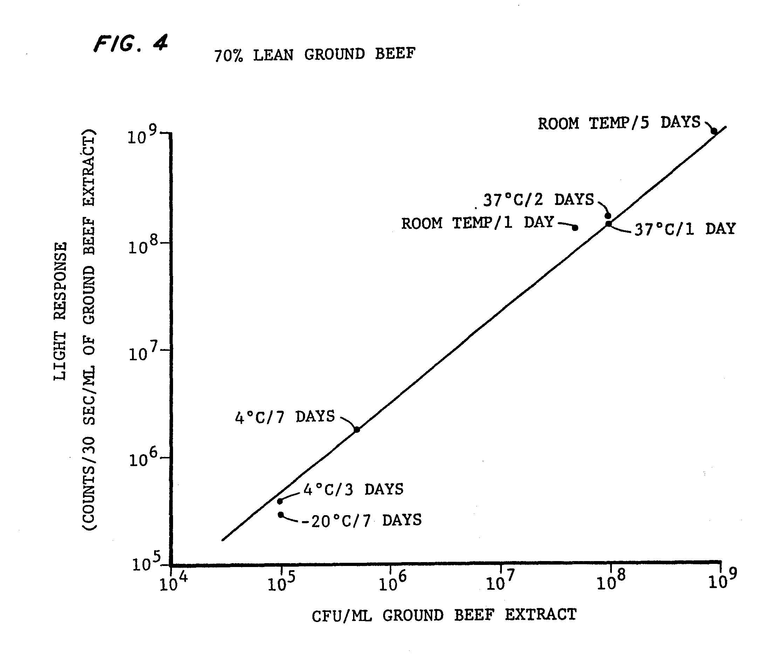

Results from quantitating bacteria in ground beef in the multiple filtration system of the present invention in conjunction with bioluminescent assay are shown in Fig. 3 for fresh meat and Fig. 4 for meat samples spoiled at different conditions. There is a clear linear relationship between the bacteria CFU in the sample and the light response recovered from the 0.4 micron pore size filter. It is interesting to observe that the luminescence response obtained has good correlation to the viable cell count of the meat sample.

Table 2

| Sample | Spoilage | Filter Light Response | | | | |
|---|---|---|---|---|---|---|
| | | PC/25/8 | PC/25/1 | PC/13/0.4 | SUM | CFU X $10^6$/gm rinse |
| 1. Whole Walleye Pike | 4°C/1day | 0.05 | 0.24 | 0.21 | 0.5 | 4.94 |
| 2. Whole Walleye Pike | Rm/1day | 1.51 | 2.80 | 26.43 | 30.74 | 153 |
| 3. Ocean Perch Filet | 4°C/1day | 0.01 | 0.01 | 0.01 | 0.03 | 0.40 |
| 4. Ocean Perch Filet | Rm/1day | 0.71 | 8.92 | 20.02 | 29.65 | 1014 |

## Example 5

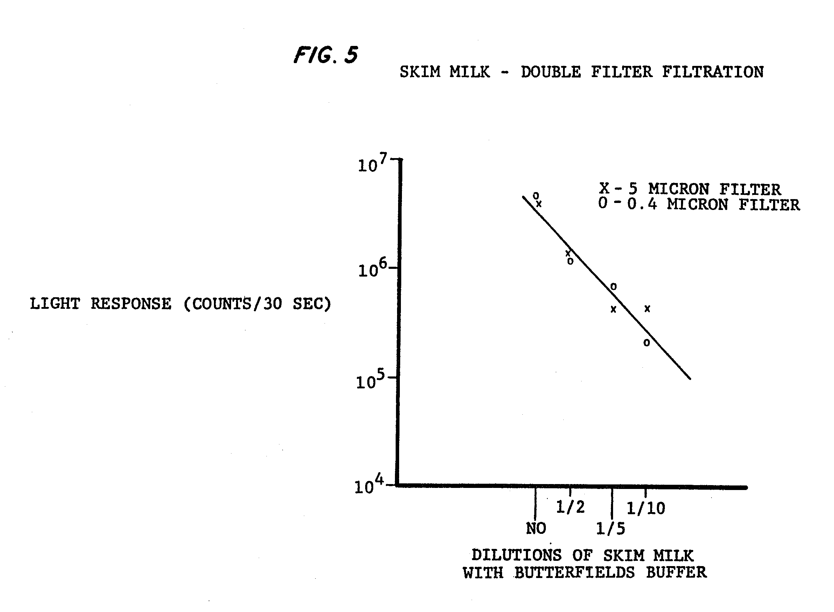

There are three or four different types of microorganisms present in milk, predominantly streptococci and lactobacilli. When passing different dilutions of skim milk through the multiple filter system of the present invention there is a correlation between the light response on filters (sum of 5 micron and 0.4 micron) and the dilution of the sample. See Fig. 5. Since the mammillian cells present in the milk sample will also be of interest, filters with pore size larger than 5 microns may be used to retain and quantitate the mammillian cells.

## Example 6

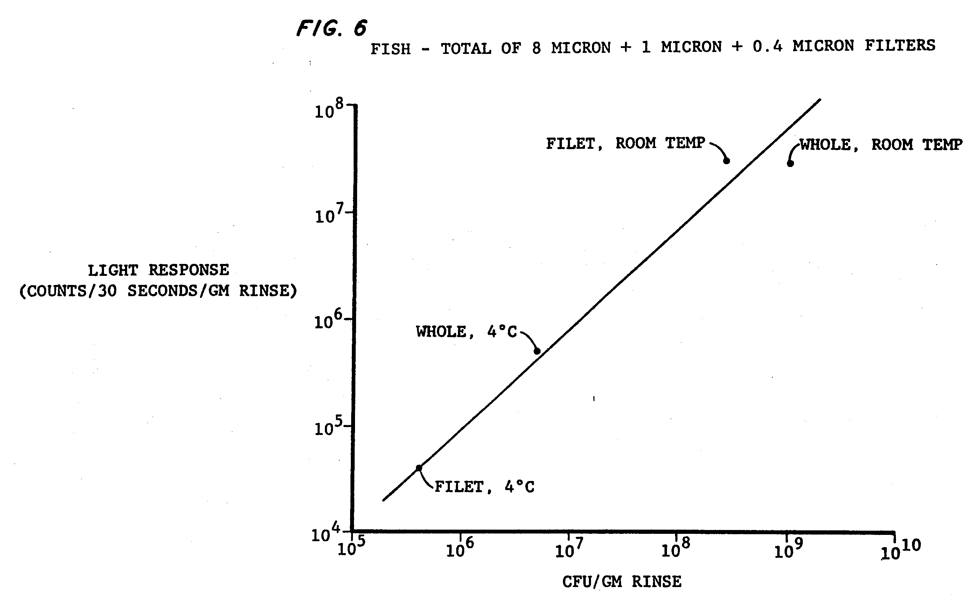

An experiment was carried out using buffer to extract surface contamination of a whole fish or fish filet spoiled at different conditions. The extraction sample is then processed through multiple filtration. The distribution of light response on each filter and the correlation of light with plate count are given in Table 2 and Fig. 6, respectively. (In the Table PC means polycarbonate, the first number is the mm diameter of the filter and the second number the micron pore size of the filter). Over half of the light response was obtained from the PC/13/0.4 filter indicating that most contaminating microorganisms of the spoiled fish samples have size between 1 micron and 0.4 micron. The CFU of the aerobic plate count ranges from $10^5$-$10^9$ cells per gram of fish rinse. The number of CFU correlates very well with the sum of light response obtained from all three filters. Zero CFU found in the end filtrate proves that there are no microorganisms escaping the 0.4 micron filter.

## Example 7

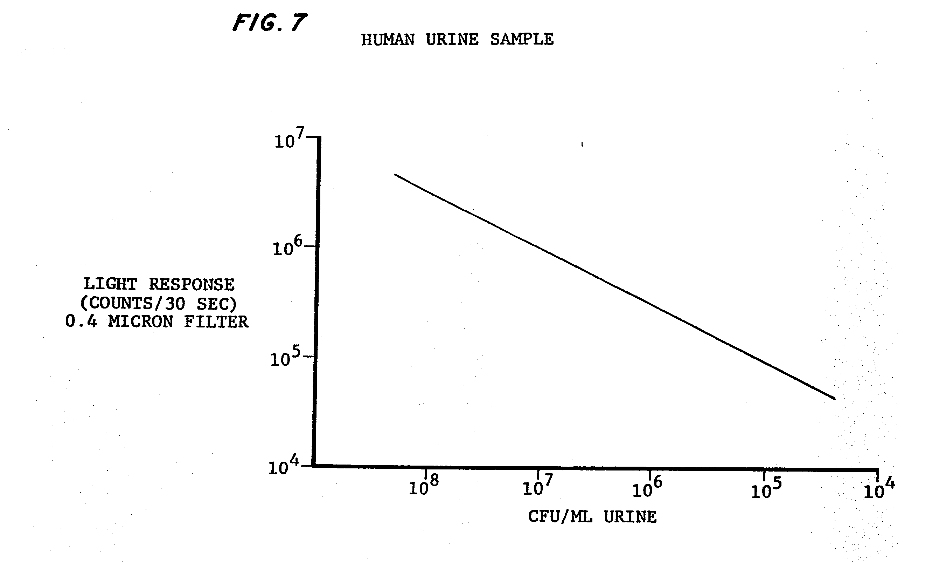

Results from a spiked human urine speciman is seen in Fig. 7. Human urine samples were spiked with different concentrations of pure cultures of E. coli and the yeast saccharomyces cerevisiae. 15 ml of the spiked sample were put through the multiple filtration technique. The correlation between the CFU from the sample and the light response from all filters is depicted in Fig. 7. This result demonstrated that multiple filtration technique can be used to filter straight urine and to quantitate microorganisms in the range of $10^5$ to $10^8$ CFU/ml urine.

### Table 3

| Time (min) | Counts X $10^5$ (30 sec) |
|---|---|
| 0 | 1.31 |
| 1 | 1.29 |
| 2 | 1.34 |
| 3 | 1.37 |
| 4 | 1.21 |

## Example 8

A speed of approximately 2.5 milliliters per minute appears to be optimal for pushing the sample through the system. During a 4 minute filtration of a 10 milliliter sample no appreciable precipitation of cells from the sample suspension was seen, as shown by the results given in Table 3. The total dead volume of the double filter system is approximately 1.8 milliliters which should be taken into account when counting cells. The membrane support screen itself will hold approximately 0.4 milliliter of liquid.

A clean filter devoid of free ATP or ATP containing entities accounts for the high resolution of this system. It is prefered to use sterile filters which are rinsed with sterile water to remove any free ATP and autoclave the whole filtering system.

The filter holders can be made of Teflon, polycarbonate or stainless steel. The sealing O-rings can be made of Teflon, ethelyne, propylene, or Viton A. As the membrane filter, as mentioned above polycarbonate, polyester, mixed esters of cellulose, and cellulose nitrate can be used although it must be kept in mind that all have different chemical capability and heat resistance, for example when autoclaved. Also, most of them are not stable under exposure to methanol, for example, for sterilization.

The use of prefilters is optional with the system and does appear to facilitate filtration by removing large particles thus minimizing interference in subsequent filtration.

Although this invention has been shown and described with respect to a prefered embodiment, it will be understood by those skilled in the art that various changes in form and detail thereof may be made without departing from the spirit and scope of the claimed invention.

## Claims

1.  A method of separating microbial ATP, non-microbial ATP, and extra-cellular ATP, comprising passing a sample containing said ATP upwards through a series of filter membranes of decreasing pore size, all membranes permeable to the extra-cellular ATP, at least one filter membrane impermeable to cells containing the microbial ATP, and at least one filter membrane impermeable to cells containing non-microbial ATP, the filter membrane impermeable to non-microbial ATP being upstream of the filter membrane impermeable to the microbial ATP.

2.  A method of separating a plurality of cell types contained in a single sample comprising passing the sample upwards through a series of filter membranes of decreasing pore size, each filter having a different pore size from the other filters and each filter sequentially being permeable to all but one of the unicellular organisms.

3.  A method of seperating and measuring cellular ATP and extra-cellular ATP comprising passing a sample containing said ATP uowards through a series of filter membranes of decreasing pore size, all membranes permeable to the extra-cellular ATP, at least one filter membrane impermeable to cells containing the cellular ATP, treating the impermeable filter membrane containing the cells sequentially with lysing agent to release the adenosime triphosphate from the cells and a luminescent reagent to react with the released ATP thereby producing a luminescent response directly on the filter membrane and measuring the luminescent response with a luminometer.

4. A method of isolating substantially pure bacteria cells from a sample also containing non-microbial soluble interferences comprising passing a sample containing said cells upwards through a series of filter membranes of decreasing pore size, all membranes permeable to the interferences and at least one filter membrane impermeable to the cells.

5. A method of cell enrichment of a specific cell type comprising concentrating the specific cell type from a sample containing a plurality of cell types by passing said cell sample containing the plurality of cell types upwards through a series of filter membranes of decreasing pore size, at least one filter membrane being impermeable to the specific cell type.

**FIG. 1**

MIXED PURE CULTURE - YEAST, E. COLI, P. AERUGINOSA, S. AUREUS - DOUBLE FILTER FILTRATION

LIGHT RESPONSE (COUNTS/30 SEC) ON 0.4 MICRON FILTER

MIXED BACTERIA CULTURE (CFU/10 ML)

FIG. 2

MIXED E. COLI AND YEAST CULTURE -
DOUBLE FILTER FILTRATION

LIGHT RESPONSE (COUNTS/30 SEC)

5 MICRON
PORE SIZE
FILTER (YEAST)

0.4 MICRON
PORE SIZE
FILTER (E.COLI)

MIXED CULTURE (CFU/10 ML)

2/7

0126019

FIG. 3  FRESH GROUND BEEF HOMOGENATE -
DOUBLE FILTER FILTRATION

LIGHT RESPONSE
(COUNTS/30 SEC)
ON
0.4 MICRON FILTER

CFU/10 ML

FIG. 4    70% LEAN GROUND BEEF

*Figure: Graph of LIGHT RESPONSE (COUNTS/30 SEC/ML OF GROUND BEEF EXTRACT) vs CFU/ML GROUND BEEF EXTRACT. Data points labeled: ROOM TEMP/5 DAYS, 37°C/2 DAYS, ROOM TEMP/1 DAY, 37°C/1 DAY, 4°C/7 DAYS, 4°C/3 DAYS, -20°C/7 DAYS.*

**FIG. 5**

SKIM MILK - DOUBLE FILTER FILTRATION

X - 5 MICRON FILTER
0 - 0.4 MICRON FILTER

LIGHT RESPONSE (COUNTS/30 SEC)

$10^7$
$10^6$
$10^5$
$10^4$

1/2    1/10
NO      1/5

DILUTIONS OF SKIM MILK
WITH BUTTERFIELDS BUFFER

FIG. 6

FISH - TOTAL OF 8 MICRON + 1 MICRON + 0.4 MICRON FILTERS

LIGHT RESPONSE
(COUNTS/30 SECONDS/GM RINSE)

FILET, ROOM TEMP

WHOLE, ROOM TEMP

WHOLE, 4°C

FILET, 4°C

CFU/GM RINSE

FIG. 7

HUMAN URINE SAMPLE

0126019